# EUROPÄISCHE PATENTSCHRIFT

(11) **EP 1 462 162 B1**
(45) Veröffentlichungstag und Bekanntmachung des Hinweises auf die Patenterteilung: **26.04.2006**
(21) Anmeldenummer: 04007200.1
(22) Anmeldetag: 25.03.2004
(51) Int. Cl.: B01J 19/02, C07C 2/00, B01J 19/00, B01J 12/00

(54) **Verfahren zum Scale-up eines Reaktors zur Durchführung einer Hochtemperaturreaktion, Reaktor und Verwendung**
Process to scale-up a reactor to obtain a high-temperature reaction, reactor and use
Procédé pour scale-up un réacteur pour la réalisation une réaction à haute température, réacteur et utilisation

(30) Priorität: 26.03.2003 DE 10313528
(43) Veröffentlichungstag der Anmeldung: 29.09.2004
(73) Patentinhaber: BASF Aktiengesellschaft, 67056 Ludwigshafen (DE)
(72) Erfinder: Bartenbach, Bernd, Dr., 67117 Limburgerhof (DE); Ehrhardt, Kai Rainer, Dr., 67063 Ludwigshafen (DE)
(74) Vertreter: Isenbruck, Günter

(56) Entgegenhaltungen:
- WO-A-01/37984
- US-A- 2 679 543
- US-A- 2 765 358
- US-A- 3 287 434
- US-A- 3 438 741
- US-A- 3 542 894

## Beschreibung

Die Erfindung betrifft ein Verfahren zum Scale-up eines Reaktors, einen Reaktor zur Durchführung einer Hochtemperaturreaktion sowie eine Verwendung.

In der chemischen Industrie werden neue Verfahren in der Regel zuerst im Labor beziehungsweise im Technikumsmaßstab durchgeführt und getestet. Die Übertragung auf die Größe des Produktionsmaßstabes ist nicht immer problemlos, da sich durch geänderte Strömungsführung der einzelnen Stoffe häufig ein anderes Verhalten einstellt als bei den Labor- bzw. Technikumsversuchen. Ein weiterer Anwendungsbereich besteht in der Vergrößerung bereits vorhandener Produktionsanlagen zur Durchsatzvergrößerung.

Als Hochtemperaturreaktionen werden in der Regel Reaktionen bezeichnet, die bei einer Temperatur oberhalb von 800°C stattfinden. Als kurze Verweilzeiten werden vorliegend Verweilzeiten im Millisekunden-Bereich, insbesondere im Bereich von etwa 1 bis 100 ms verstanden. Analog wird als schnelle Abkühlung eine Abkühlung im Millisekunden-Bereich, insbesondere im Bereich von etwa 1 bis 100 ms, definiert.

Reaktoren, in denen eine Hochtemperaturreaktion mit kurzer Verweilzeit stattfindet und das Reaktionsgemisch anschließend in einem Quenchbereich schnell abgekühlt wird, werden zum Beispiel für die Acetylenherstellung eingesetzt.

Die im heutigen Produktionsmaßstab eingesetzten Reaktoren zur Acetylenherstellung zeichnen sich durch eine zylinderförmige Geometrie des Reaktionsraumes aus. Ein derartiger Reaktor ist beispielsweise in DE-A 44 22 815 beschrieben. Die vorgemischten Reaktionsstoffe werden über einen Diffusor rückvermischungsfrei einem Brennerblock zugeführt. Der Brennerblock hat einen kreisförmigen Querschnitt und ist von Kanälen durchsetzt. Das Reaktionsgemisch wird über die Kanäle einem Reaktionsraum mit ebenfalls kreisförmigem Querschnitt zugeführt. Im Reaktionsraum reagiert das Reaktionsgemisch unter Flammenbildung in einer acetylenbildenden partiellen Oxidationsreaktion. An den Reaktionsraum schließt sich ein Quenchbehälter größeren Querschnitts an, in dem Quenchdüsen auf einem oder mehreren Quenchverteilerringen installiert sind, die Wasser oder Öl zerstäuben und näherungsweise senkrecht zur Hauptströmungsrichtung in das den Feuerraum verlassende Reaktionsgas eindüsen. Die Aufgabe dieses direkten Wasser- oder Ölquenches ist es, das Reaktionsgemisch extrem schnell abzukühlen, so dass Folgereaktionen, wie insbesondere der Abbau des gebildeten Acetylens, eingefroren werden. Dabei ist die Reichweite und Verteilung der Quenchstrahlen idealerweise so bemessen, dass eine möglichst homogene Temperaturverteilung in möglichst kurzer Zeit erreicht wird.

Eine Vergrößerung des Reaktors ist problematisch, da mit zunehmendem Durchmesser des zylinderförmigen Reaktionsraumes und des zylinderförmigen Quenchbereiches die Homogenität der Quenchwirkung immer schwieriger gewährleistet werden kann. Das liegt daran, dass die Eindringtiefe der Spraystrahlen und die Tröpfchengröße bei der Zerstäubung direkt zusammenhängen. Das heißt, dass die für eine schnelle Verdampfung und damit eine gute Quenchwirkung erforderlichen kleinen Tröpfchen nur begrenzte Eindringtiefen erreichen, da die kleinen Tröpfchen aufgrund des geringen Impulses frühzeitig abgelenkt werden. Hierdurch treten Inhomogenitäten bei der Quenchwirkung auf, die den Acetylenabbau in den heißeren Strähnen begünstigen. Hieraus resultieren die in der Vergangenheit beobachteten Unterschiede in der Acetylenausbeute zwischen den kleineren, älteren Bauformen der Reaktoren und den großen heute im Einsatz befindlichen Reaktoren, die eine Ausbeute von etwa 25 Tagestonnen liefern. Die kleineren Brenner erreichen Ausbeuten, die in etwa 10 % - relativ zur Ausbeute der großen Brenner - höher liegen.

Es war die Aufgabe der Erfindung, eine Brennergeometrie zu finden, die bei einer Maßstabsvergrößerung keine Ausbeuteeinbußen verursacht, um dadurch mit weniger Produktionssträngen mit jeweils größeren Einheiten auszukommen beziehungsweise bei existierenden größeren Einheiten mit einem vorgegebenen Maßstab eine Ausbeute- und damit Kapazitätssteigerung zu erreichen.

Die Aufgabe wird gelöst durch ein Verfahren zum Scale-up eines Reaktors mit Zuführung eines Reaktionsgemisches über Kanäle eines Brennerblockes in einen Reaktionsraum, wobei im Reaktionsraum eine Hochtemperaturreaktion mit kurzer Verweilzeit stattfindet und das Reaktionsgemisch anschließend in einem Quenchbereich schnell abgekühlt wird, dadurch gekennzeichnet, dass man für eine Durchsatzvergrößerung den Innendurchmesser des Reaktors vergrößert, wobei man den Übergang des Reaktionsraums in den Quenchbereich in Form eines Spaltes ausbildet, der auf eine Breite im Bereich von 2 bis 200 mm begrenzt ist.

Bevorzugt ist der Übergang des Reaktionsraumes in den Quenchbereich auf einen Spalt mit einer Breite im Bereich von 50 bis 150 mm begrenzt.

Mit der hier dargestellten erfindungsgemäßen Lösung werden die Nachteile der Vergrößerung des zylinderförmigen Querschnittes bezüglich der realisierbaren Quenchwirkung vermieden, indem von der zylinderförmigen Geometrie auf eine spaltförmige Geometrie übergegangen wird. Hierbei wird der Spalt so ausgeführt, dass eine Wärmeabfuhr durch direkte Wassereindüsung von einer oder von beiden Seiten des Spaltes bei geringen Strahlreichweiten und feinsten Sprays sehr effektiv und homogen gelingt.

Bevorzugt wird dieser Spalt als Ringspalt ausgebildet, damit vorangehende und nachgeschaltete Anlagenteile, die in der Regel einen zylinderförmigen Querschnitt aufweisen, leichter eingebunden werden können.

Gegenstand der Erfindung ist auch ein Reaktor mit Zuführung eines Reaktionsgemisches über Kanäle eines Brennerblockes in einen Reaktionsraum, wobei im Reaktionsraum eine Hochtemperaturreaktion mit kurzer Verweilzeit stattfindet und das Reaktionsgemisch anschließend in einem Quenchbereich schnell abgekühlt wird, wobei der Reaktor dadurch gekennzeichnet ist, dass man den Übergang des Reaktionsraumes in den Quenchbereich in Form eines Spaltes ausbildet. Bevorzugt ist der Spalt auf eine Breite im Bereich von 2 bis 200 mm, insbesondere im Bereich von 50 bis 150 mm, begrenzt.

In einer weiteren bevorzugten Ausführungsvariante ist auch der Reaktionsraum in Form eines Ringspaltes ausgebildet.

Die spaltförmige, vorzugsweise ringspaltförmige Geometrie des Überganges vom Reaktionsraum in den Quenchbereich ermöglicht eine Eindüsung des Quenchmediums, zum Beispiel Wasser oder Öl, entweder von einer Seite des Spaltes oder von beiden Seiten des Spaltes.

Die zur Eindüsung erforderlichen Quenchdüsen können dabei in einer oder in mehreren Reihen, zum Beispiel versetzt oder linear angeordnet sein.

Bevorzugt bewirkt man im Quenchbereich ein direktes Quenchen durch Einbringen eines Kühlmediums über Quenchdüsen, die radial oder tangential zur Hauptströmungsrichtung des Reaktionsgemisches im Reaktor angeordnet sind, wobei bei einem mehrstufigen Einbringen von Kühlmedium eine gegenläufige Anstellung der Quenchdüsen bevorzugt ist. Dabei ist neben einer radialen oder tangentialen Orientierung der Quenchdüsen zur Hauptströmungsrichtung des Reaktionsgemisches im Reaktor, in der Querschnittsebene senkrecht zur Hauptströmungsrichtung betrachtet, auch eine Orientierung in einem beliebigen Winkel zur Hauptströmungsrichtung möglich.

Als Quenchdüsen können Druck-Zerstäuber oder Zerstäuber, die durch ein Hilfsmedium gestützt werden, zum Beispiel Dampf, eingesetzt werden.

Die schnelle Abkühlung des Reaktionsgemisches im Quenchbereich kann man bevorzugt durch direktes oder indirektes Quenchen bewirken, d.h. neben der direkten Abkühlung durch Eindüsen eines Mediums ist auch eine indirekte Abkühlung in einem Wärmeübertrager möglich.

Das direkte Quenchen des Reaktionsgemisches im Quenchbereich kann man durch ein- oder mehrstufiges Einmischen eines Kühlmediums in das Reaktionsgemisch, bevorzugt über einen oder mehrere Ringverteiler, bewirken.

Es ist möglich, das direkte Quenchen des Reaktionsmediums im ringspaltförmig ausgebildeten Quenchbereich durch direktes Einmischen von Kühlmedium in den ringspaltförmig ausgebildeten Quenchbereich von außen und/oder von innen zu bewirken.

Der Quenchbereich ist bevorzugt fluchtend in Richtung der Längsachse des Reaktionsraumes ausgebildet, insbesondere als Spalt, besonders bevorzugt als Ringspalt.

Um die ringspaltförmig ausgebildeten Bauteile in rohrförmige Anschlussquerschnitte einzufügen, sollte der ringspaltförmig ausgebildete Kanal über eine sich allmählich erweiternde, zum Beispiel in Form eines Rotationsellipsoides ausgebildete Nabenversperrung verfügen. Die Nabenversperrung kann massiv oder bevorzugt als innengekühlter Hohlkörper ausgebildet sein. Die Nabenversperrung ist weitestmöglich strömungsablösungsfrei zu gestalten.

Alternativ ist eine aerodynamische Anpassung der Nabenversperrung durch Spray- oder Gasstrahlen in axialer Richtung denkbar.

Die Nabenversperrung kann gekühlt oder ungekühlt ausgeführt werden.

Im Bereich des Brennerblockes kann der ringspaltförmige Querschnitt mit Bohrungen, die in Längsachse des Reaktionsraumes ausgerichtet sind, ausgeführt werden. Dabei sind die Bohrungen zum Beispiel in mehrfachen konzentrischen Kreisringen oder in hexagonaler Struktur angeordnet. Neben den parallel zur Längsachse des Reaktionsraumes ausgerichteten Kanälen können auch Kanäle für die Zuführung des Reaktionsgemisches und Kanäle für die Zuführung von zusätzlichem Sauerstoff oder von Reaktionshilfsstoffen in einem beliebigen Winkel zur Längsachse des Reaktionsraumes ausgerichtet sein.

Neben der Herstellung von Acetylen lässt sich das Prinzip der Ringspaltgeometrie auch auf die Herstellung anderer Produkte, die einen intensiven indirekten oder direkten Quench der Reaktion benötigen, übertragen.

Um Koks- und Russablagerungen zu vermeiden, ist es bevorzugt, alle den Reaktionsraum begrenzenden Oberflächen aus einer bei Reaktionstemperatur beständigen Feuerfestkeramik mit einem Aluminiumoxid-Anteil von mindestens 80% auszubilden. Hierbei ist der Werkstoff des Grundkörpers, auf den die Feuerfestkeramik aufzubringen ist, nicht erfindungswesentlich. Bevorzugt handelt es sich hierbei um metallische Werkstoffe.

Eine Beständigkeit der Keramik gegen die bei den Hochtemperaturreaktionen auftretenden Temperaturen von über 800°C, insbesondere über 1300°C wird durch einen Aluminiumoxidanteil von mindestens 80% Gew.-%, bevorzugt von mindestens 95 Gew.-% und besonders bevorzugt von mindestens 96 Gew.-%, erreicht.

Die Auskleidung des Reaktionsraumes erfolgt in einer ersten Ausführungsvariante durch Ausmauern mit der Feuerfestkeramik in Form von Steinen oder Blöcken.

In einer zweiten Ausführungsvariante wird die Feuerfestkeramik in Form einer Gieß- oder Stampfmasse in den Reaktionsraum eingebracht und dort anschließend verdichtet, getrocknet und gebrannt. In einer bevorzugten Ausführungsform wird die als Gieß- oder Stampfmasse in den Reaktionsraum eingebrachte Feuerfestkeramik durch die Hochtemperaturreaktion gebrannt.

Die Feuerfestkeramik, mit der der Reaktionsraum ausgekleidet ist, weist vorteilhaft eine Dicke im Bereich von 7 bis 30 cm, bevorzugt eine Dicke im Bereich von 8 bis 10 cm, auf. Zusätzlich kann eine Hinterisolierung aus einer Keramik mit besonders guten wärmeisolierenden Eigenschaften erfolgen.

Ein Vorteil der Auskleidung mit einer Feuerfestkeramik ist, dass die Auskleidung thermisch isolierend wirkt. Aus diesem Grund braucht die Wand des Reaktionsraumes nicht mehr gekühlt zu werden, was zu einer Einsparung an Kühlmedium führt. Ein weiterer Vorteil ist, dass durch ausreichend gute Feuerfestisolierung selbst unter extremen Bedingungen, d.h. bei sehr hohen Temperaturen und starker Russbildung die Russabscheidung und damit die Bildung von Koks verhindert werden kann. Hierdurch lässt sich die mechanische Stochereinrichtung und deren aufwendige Wartung einsparen. Zudem entfallen durch die Einsparung der mechanischen Stochereinrichtung durch Stocherfehler ausgelöste Betriebsunterbrechungen. Schließlich wird die Materialbelastung durch die erosive Wirkung des abgestocherten Kokses auf die nachgeschalteten Anlageteile, wie Pumpen, Wärmeübertrager und Rohrleitungen, drastisch reduziert.

Gegenstand der Erfindung ist auch die Verwendung des vorstehend beschriebenen Verfahrens oder des vorstehend beschriebenen Reaktors zur Herstellung von Acetylen durch partielle Oxidation von Kohlenwasserstoffen mit Sauerstoff.

Die Erfindung wird im Folgenden anhand einer Figur sowie von Anwendungsbeispielen näher erläutert.

Es zeigen im Einzelnen:
- Figur 1: eine Ausführungsform eines erfindungsgemäß ausgebildeten Reaktors zur Acetylenherstellung,
- Figur 2: einen Ausschnitt aus einem Reaktor, bestehend aus Brennerblock, Reaktionsraum und Quenchbereich,
- Figur 3: einen Schnitt durch einen ringspaltförmig ausgebildeten Brennerblock,
- Figur 4: Temperaturverläufe in einem ringspaltförmig ausgebildeten Quenchbereich mit halbellipsoidaler Nabenversperrung.

In den Figuren bezeichnen gleiche Bezugszeichen gleiche oder entsprechende Merkmale.

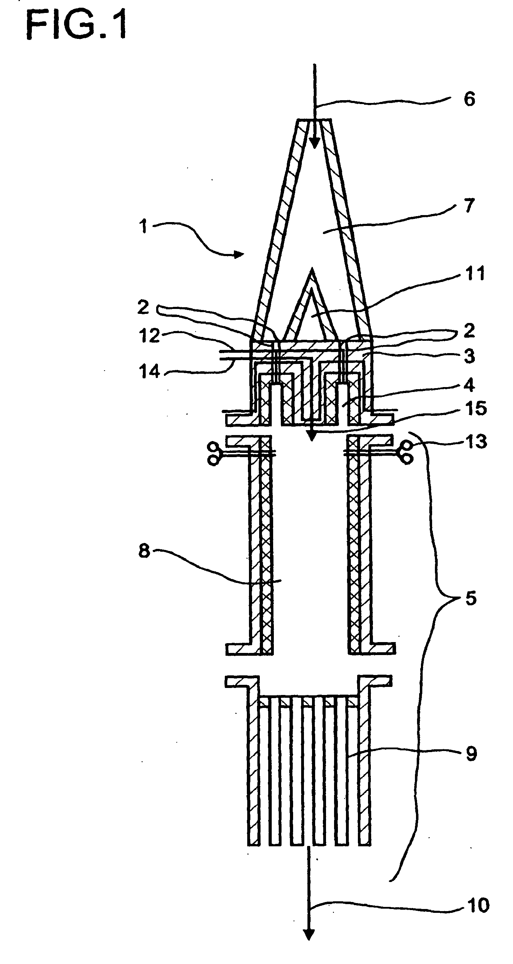

Figur 1 zeigt einen Reaktor 1 zur Acetylenherstellung mit einem ringspaltförmig ausgebildeten Reaktionsraum 4. Das Reaktionsgemisch wird über eine Aufgabestelle 6 durch einen Diffusor 7 über Kanäle 2 in einem Brennerblock 3 einem Reaktionsraum 4 zugeführt. Im Reaktionsraum 4 findet eine Hochtemperaturreaktion mit kurzer Verweilzeit statt. Das Reaktionsgemisch wird anschließend in einem Quenchbereich 5 schnell abgekühlt. Die Mischung aus Reaktionsgemisch und Quenchmedium durchströmt einen Quenchbehälter 8 und gelangt in einen Wärmeübertrager 9, der zur Ausnutzung der Restwärme des Reaktionsgemisches genutzt werden kann. Aus dem Wärmeübertrager 9 gelangt das Reaktionsgemisch über einen Ablauf 10 zu weiteren Prozessschritten.

Um eine störungsfreie Strömung des Reaktionsgemisches aus dem Diffusor 7 in die Kanäle 2 des Brennerblockes 3 zu gewährleisten, befindet sich im Diffusor 7 eine Nabenversperrung 11. Die Nabenversperrung 11 ist einer bevorzugten Ausführungsvariante als Kegel oder Halbellipsoid gestaltet.

Der Brennerblock 3 umfasst neben den Kanälen 2 für das Reaktionsgemisch, das durch den Diffusor 7 dem Reaktionsraum 4 zugeführt wird, Zusatzkanäle 12 für Reaktionshilfsstoffe oder zusätzlichen Sauerstoff.

Das Quenchmedium, beispielsweise Wasser oder Öl, wird über einen ringförmigen Quenchverteiler 13 mit Einspritzdüsen, die ringförmig am Quenchbereich 5 angeordnet sind, zugeführt. Neben den Quenchdüsen, die über den äußeren Quenchverteiler 13 versorgt werden, kann auch über innere Quenchdüsen 15, die über eine Leitung 14 versorgt werden, Quenchmedium zur Abkühlung in den Quenchbereich 5 eingesprüht werden.

Zur Vermeidung von Koks- und Russanbackungen ist der Reaktionsraum 4, der Quenchbereich 5 und der Quenchbehälter 8 mit einer Feuerfestkeramik 16 ausgekleidet.

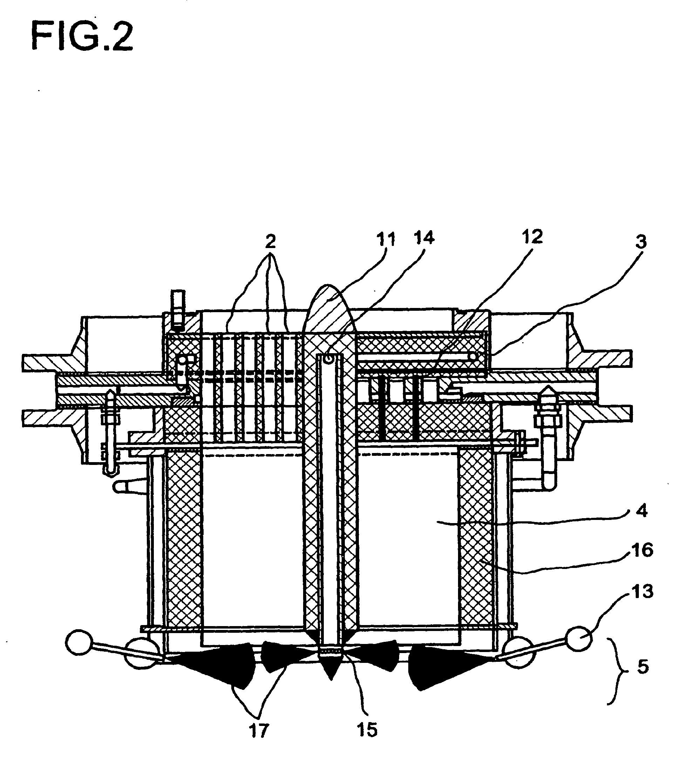

Figur 2 zeigt einen Ausschnitt aus einem Reaktor 1, der den Brennerblock 3, den Reaktionsraum 4 und den Quenchbereich 5 umfasst. Im Unterschied zu Figur 1 ist in Figur 2 die Nabenversperrung 11 nicht in Form eines Kegels, sondern in Form eines Halbellipsoiden ausgebildet. Weiterhin zeigt Figur 2 die Sprühstrahlen 17 des Quenchmediums. Die Sprühstrahlen 17 sind von den äußeren Quenchdüsen, die über den Quenchverteiler 13 versorgt werden, nach innen gerichtet und von den inneren Quenchdüsen 15, die über die Leitung 14 versorgt werden, nach außen gerichtet.

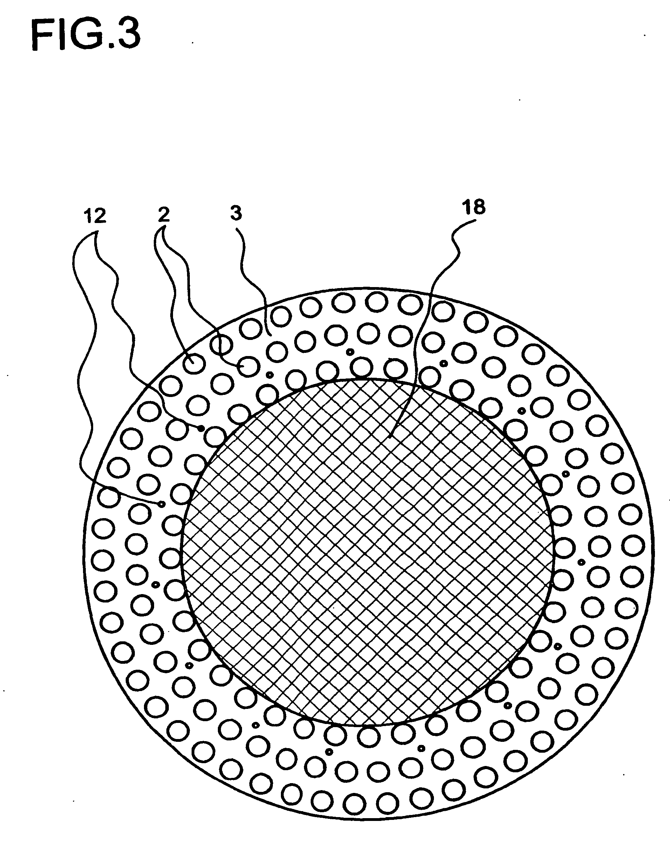

Figur 3 ist die Draufsicht auf einen erfindungsgemäß ausgestalteten Brennerblock zu entnehmen. Lm Brennerblock 3 sind die Kanäle 2 konzentrisch angeordnet. Neben der konzentrischen Anordnung könnten die Kanäle auch hexagonal oder in weiteren Anordnungen angeordnet sein. Zusätzlich zu den Kanälen 2 lassen sich die Zusatzkanäle 12, die hier ebenfalls konzentrisch angeordnet sind, erkennen. Der innere Bereich 18 des Brennerblocks 3 ist hier durch eine Feuerfestkeramik verschlossen. Neben der vollständig geschlossenen Ausführungsvariante, wie sie hier dargestellt ist, kann der innere Bereich 18 des Brennerblockes 3 auch die Leitung 14 für die Zuführung des Quenchmediums zu den inneren Quenchdüsen 15 enthalten. Weiterhin kann der innere Bereich 18 des Brennerblocks 3 auch als Hohlzylinder, zum Beispiel zum Zwecke der Kühlung, ausgebildet sein.

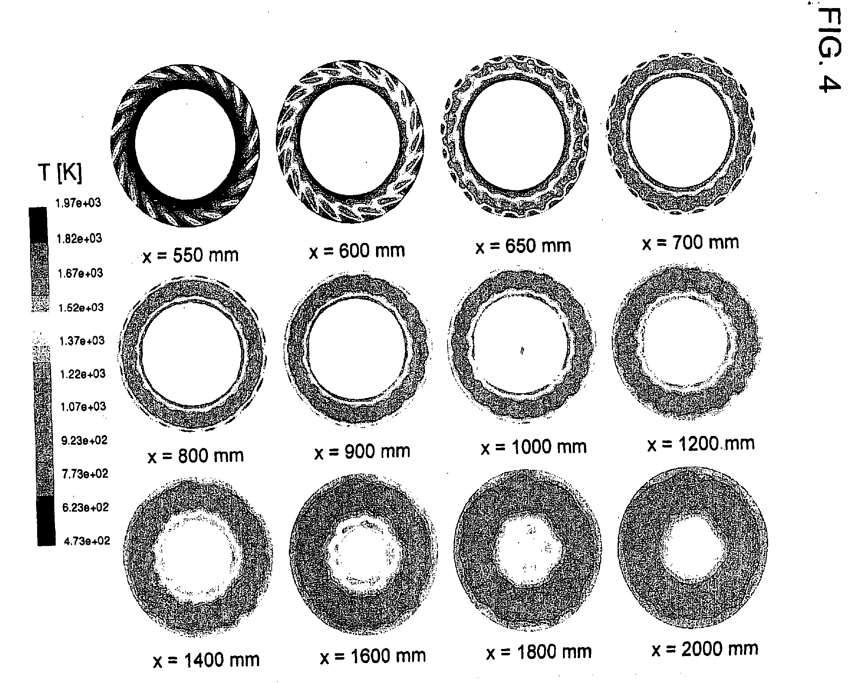

Figur 4 zeigt die mit der kommerziellen Software Fluent® berechneten Temperaturverläufe entlang des Quenchbereiches mit einer halbellipsoidalen Nabenversperrung.

An der Stelle x = 550 mm wird Quenchmedium mit ringförmig angeordneten Quenchdüsen in tangentialer Richtung im Uhrzeigersinn in den als Ringspalt ausgeführten Quenchbereich eingesprüht. Im weiteren Verlauf lässt sich erkennen, wie die Temperatur im Bereich der Quenchstrahlen abnimmt. Bei x = 650 mm wird an einer zweiten Stelle Quenchmedium tangential gegen den Uhrzeigersinn in den ringspaltförmig ausgebildeten Quenchbereich eingesprüht. Die weiteren Bilder zeigen, wie sich die Temperatur im weiteren Verlauf entlang des Quenchbereiches verändert. Hierbei ist deutlich zu sehen, dass bei einer Position x = 1800 mm nicht mehr die Richtung der Sprühstrahlen zu erkennen ist, sondern sich von außen nach innen eine ringförmig gleichmäßige Temperaturverteilung einstellt. Hierbei weist die Temperatur im Inneren des Quenchbereiches noch eine etwas höhere Temperatur auf als im äußeren Bereich.

### Anwendungsbeispiele

Der Einfluss der Spaltgeometrie auf die Acetylenausbeute wurde an einem gemäß Figur 1 ausgebildeten Reaktor untersucht. Der Außendurchmesser des Ringspaltes der eingesetzten Versuchsapparatur betrug 720 mm, der Innendurchmesser 480 mm. Daraus ergab sich eine Spaltweite von 120 mm. Der in die Versuchsapparatur eingesetzte Brennerblock war gemäß Figur 3 ausgebildete und wies 126 Bohrungen verteilt auf 3 Kreisringe auf. Zur Abkühlung des Reaktionsgemisches wurden 50 Quenchdüsen am äußeren Umfang des Quenchbereiches mit Wasser als Quenchmedium verteilt angebracht.

Als Reaktionsstoffe wurden Sauerstoff und Erdgas in einem Verhältnis von Sauerstoff zu Erdgas von 0,59 eingesetzt. Die Reaktionsstoffe wurden in einer Vorheizung auf 600 °C erhitzt, bevor sie dem Reaktionsraum zugeführt wurden. Das bei der Reaktion entstandene Reaktionsgemisch enthielt einen Anteil von 9,4 % Acetylen. Neben dem Acetylen sind 3,4 % Kohlendioxid, 5,7 % Methan, 24 % Kohlenmonoxid und 56 % Wasserstoff enthalten. Damit beträgt die Ausbeute an Acetylen hier ca. 32,5 % auf Kohlenstoff bezogen.

Im Vergleich dazu erhält man in einem konventionellen Brenner mit Zylindergeometrie bei gleichen Randbedingungen etwa 7,5 - 8,5 % Acetylen im Reaktionsgemisch. Eine Beispielmessung im laufenden Betrieb ergab einen Anteil von 3,4 % Kohlendioxid, 8,1 % Acetylen, 5,5 % Methan, 25,2 % Kohlenmonoxid und 56,4 % Wasserstoff im Reaktionsgemisch. Damit betrug die Ausbeute hier ca. 29,5 % auf Kohlenstoff bezogen.

### Bezugszeichenliste

- 1: Reaktor
- 2: Kanäle
- 3: Brennerblock
- 4: Reaktionsraum
- 5: Quenchbereich
- 6: Aufgabestelle
- 7: Diffusor
- 8: Quenchbehälter
- 9: Wärmeübertrager
- 10: Ablauf
- 11: Nabenversperrung
- 12: Zusatzkanäle
- 13: Quenchverteiler
- 14: Leitung
- 15: innere Quenchdüsen
- 16: Feuerfestkeramik
- 17: Sprühstrahl
- 18: innerer Bereich

## Patentansprüche

1. Verfahren zum Scale-up eines Reaktors (1) mit Zuführung eines Reaktionsgemisches über Kanäle (2) eines Brennerblockes (3) in einen Reaktionsraum (4), wobei im Reaktionsraum (4) eine Hochtemperaturreaktion mit kurzer Verweilzeit stattfindet und das Reaktionsgemisch anschließend in einem Quenchbereich (5) schnell abgekühlt wird, **dadurch gekennzeichnet, dass** man für eine Durchsatzvergrößerung den Innendurchmesser des Reaktors (1) vergrößert, wobei man den Übergang des Reaktionsraums (4) in den Quenchbereich (5) in Form eines Spaltes ausbildet, der auf eine Breite im Bereich von 2 bis 200 mm begrenzt ist.

2. Verfahren nach Anspruch 1, **dadurch gekennzeichnet, dass** man den Übergang des Reaktionsraums (4) in den Quenchbereich (5) auf einen Spalt mit einer Breite im Bereich von 50 bis 150 mm begrenzt.

3. Reaktor (1) mit Zuführung eines Reaktionsgemisches über Kanäle (2) eines Brennerblockes (3) in einen Reaktionsraum (4), wobei im Reaktionsraum (4) eine Hochtemperaturreaktion mit kurzer Verweilzeit stattfindet und das Reaktionsgemisch anschließend in einem Quenchbereich (5) schnell abgekühlt wird, **dadurch gekennzeichnet, dass** der Übergang des Reaktionsraumes (4) in den Quenchbereich (5) in Form eines Ringspaltes ausgebildet ist, der bevorzugt auf eine Breite im Bereich von 2 bis 200 mm begrenzt ist, besonders bevorzugt im Bereich von 50 bis 150 mm.

4. Reaktor (1) nach Anspruch 3, **dadurch gekennzeichnet, dass** man den Reaktionsraum (4) in Form eines Ringspaltes ausbildet.

5. Reaktor (1) nach Anspruch 3 oder 4, **dadurch gekennzeichnet, dass** man die Kanäle (2) des Brennerblocks (3) in Längsachse des Reaktionsraums (4) ausrichtet.

6. Reaktor (1) nach Anspruch 3 oder 4, **dadurch gekennzeichnet, dass** man einen Teil der Kanäle (2) für die Zuführung des Reaktionsgemisches und/oder Kanäle (6) für die Zuführung von zusätzlichem Sauerstoff oder von Reaktionshilfsstoffen in einem beliebigen Winkel zur Längsachse des Reaktionsraumes (4) ausrichtet und im übrigen die Kanäle (2) des Brennerblocks (3) in Längsachse des Reaktionsraums (4) ausrichtet.

7. Reaktor (1) nach einem der Ansprüche 3 bis 6, **dadurch gekennzeichnet, dass** man den Quenchbereich (5) fluchtend in Richtung der Längsachse des Reaktionsraumes (4), bevorzugt als Spalt, besonders bevorzugt als Ringspalt, ausbildet.

8. Reaktor (1) nach einem der Ansprüche 3 bis 7, **dadurch gekennzeichnet, dass** man die schnelle Abkühlung des Reaktionsgemisches im Quenchbereich (5) durch direktes oder indirektes Quenchen bewirkt, wobei man das direkte Quenchen des Reaktionsgemisches im Quenchbereich (5) bevorzugt durch ein- oder mehrstufiges Einmischen eines Kühlmediums in das Reaktionsgemisch, besonders bevorzugt über einen oder mehrere Ringverteiler, bewirkt.

9. Reaktor (1) nach Anspruch 8, **dadurch gekennzeichnet, dass** man das direkte Quenchen des Reaktionsmediums im ringspaltförmig ausgebildeten Quenchbereich (5) durch direktes Einmischen von Kühlmedium in den ringspaltförmig ausgebildeten Quenchbereich (5) von außen und/oder von innen bewirkt.

10. Reaktor (1) nach einem der Ansprüche 3 bis 9, **dadurch gekennzeichnet, dass** man im Quenchbereich (5) ein direktes Quenchen durch Einbringen eines Kühlmediums über Quenchdüsen bewirkt, die radial oder tangential zur Hauptströmungsrichtung des Reaktionsgemisches im Reaktor (1) angeordnet sind, wobei bei einem mehrstufigen Einbringen von Kühlmedium eine gegenläufige Anstellung der Quenchdüsen bevorzugt ist.

11. Reaktor (1) nach einem der Ansprüche 3 bis 10, **dadurch gekennzeichnet, dass** alle den Reaktionsraum (4) begrenzenden Oberflächen aus einer bei Reaktionstemperatur beständigen Feuerfestkeramik mit einem Aluminiumoxid-Anteil von mindestens 80 Gew.-%, bevorzugt von mindestens 95 Gew.-%, besonders bevorzugt von mindestens 96 Gew.-%, ausgebildet sind.

12. Reaktor (1) nach einem der Ansprüche 3 bis 11, **dadurch gekennzeichnet, dass** die Feuerfestkeramik in Form von Steinen oder Blöcken oder als Gieß- oder Stampfmasse in den Reaktionsraum eingebracht und anschließend verdichtet, getrocknet und bevorzugt durch die Hochtemperaturreaktion gebrannt ist.

13. Reaktor (1) nach einem der Ansprüche 3 bis 12, **dadurch gekennzeichnet, dass** die Feuerfestkeramik eine Dicke im Bereich von 7 bis 30 cm, bevorzugt eine Dicke im Bereich von 8 bis 10 cm, aufweist.

14. Verwendung des Verfahrens nach Anspruch 1 oder 2 oder des Reaktors nach einem der Ansprüche 3 bis 13 zur Herstellung von Acetylen durch partielle Oxidation von Kohlenwasserstoffen mit Sauerstoff.

## Claims

1. A process for the scale-up of a reactor (1) having a supply of a reaction mixture via channels (2) of a burner block (3) to a reaction chamber (4), a high temperature reaction having a short residence time taking place in the reaction chamber (4) and the reaction mixture subsequently being rapidly cooled in a quench area (5), **characterized in that** for a throughput enlargement the internal diameter of the reactor (1) is enlarged, the transition from the reaction chamber (4) to the quench area (5) being designed in the form of a gap which is restricted to a width in the range from 2 to 200 mm.

2. The process according to claim 1, **characterized in that** the transition of the reaction chamber (4) to the quench area (5) is restricted to a gap having a width in the range from 50 to 150 mm.

3. A reactor (1) having a supply of a reaction mixture via channels (2) of a burner block (3) to a reaction chamber (4), a high temperature reaction having a short residence time taking place in the reaction chamber (4) and the reaction mixture subsequently being rapidly cooled in a quench area (5), **characterized in that** the transition of the reaction chamber (4) to the quench area (5) is designed in the form of an annular gap which is preferably restricted to a width in the range from 2 to 200 mm, particularly preferably in the range from 50 to 150 mm.

4. The reactor (1) according to claim 3, **characterized in that** the reaction chamber (4) is designed in the form of an annular gap.

5. The reactor (1) according to claim 3 or 4, **characterized in that** the channels (2) of the burner block (3) are aligned in the longitudinal axis of the reaction chamber (4).

6. The reactor (1) according to claim 3 or 4, **characterized in that** some of the channels (2) for the supply of the reaction mixture and/or channels (6) for the supply of additional oxygen or of reaction auxiliaries are aligned at any desired angle to the longitudinal axis of the reaction chamber (4) and otherwise the channels (2) of the burner block (3) are aligned in the longitudinal axis of the reaction chamber (4).

7. The reactor (1) according to one of claims 3 to 6, **characterized in that** the quench area (5) is constructed aligning in the direction of the longitudinal axis of the reaction chamber (4), particularly as a gap, particularly preferably as an annular gap.

8. The reactor (1) according to one of claims 3 to 7, **characterized in that** the rapid cooling of the reaction mixture in the quench area (5) is brought about by direct or indirect quenching, wherein the direct quenching of the reaction mixture in the quench area (5) is preferably brought about by single- or multistage mixing of a cooling medium into the reaction mixture, particularly preferably via one or more annular distributors.

9. The reactor (1) according to claim 8, **characterized in that** the direct quenching of the reaction medium in the quench area (5) designed like an annular gap is brought about by direct mixing of cooling medium into the quench area (5) designed like an annular gap from outside and/or from inside.

10. The reactor (1) according to one of claims 3 to 9, **characterized in that** in the quench area (5) direct quenching is brought about by introducing a cooling medium via quench nozzles which are arranged radially or tangentially to the main flow direction of the reaction mixture in the reactor (1), where in the case of a multistage introduction of cooling medium a countercurrent positioning of the quench nozzles is preferred.

11. A reactor (1) according to one of claims 3 to 10, **characterized in that** all surfaces restricting the reaction chamber (4) are formed of a fire-resistant ceramic which is stable at reaction temperature having an alumina content of at least 80% by weight, preferably of at least 95% by weight, particularly preferably of at least 96% by weight.

12. The reactor (1) according to one of claims 3 to 11, **characterized in that** the fire-resistant ceramic is introduced into the reaction chamber in the form of stones or blocks or as a cast or tamped mass and subsequently compressed, dried and preferably calcined by the high temperature reaction.

13. The reactor (1) according to one of claims 3 to 12, **characterized in that** the fire-resistant ceramic has a thickness in the range from 7 to 30 cm, preferably a thickness in the range from 8 to 10 cm.

14. The use of the process according to claim 1 or 2 or of the reactor according to one of claims 3 to 13 for the preparation of acetylene by partial oxidation of hydrocarbons using oxygen.

## Revendications

1. Procédé de détartrage d'un réacteur (1) comprenant une amenée d'un mélange réactionnel dans une chambre réactionnelle (4) par l'intermédiaire de canaux (2) d'un bloc de brûleur (3), une réaction à haute température à courte durée de séjour ayant lieu dans la chambre réactionnelle (4) et le mélange réactionnel étant ensuite rapidement refroidi dans une zone de refroidissement rapide (5), **caractérisé en ce que**, pour un agrandissement du débit, on agrandit le diamètre interne du réacteur (1), la transition de la chambre réactionnelle (4) à la zone de refroidissement rapide (5) étant réalisée sous la forme d'une fente qui est limitée à une largeur de l'ordre de 2 à 200 mm.

2. Procédé suivant la revendication 1, **caractérisé en ce qu'**on limite la transition de la chambre réactionnelle (4) à la zone de refroidissement rapide (5) à une fente ayant une largeur de l'ordre de 50 à 150 mm.

3. Réacteur (1) comportant une amenée d'un mélange réactionnel dans une chambre réactionnelle (4) par l'intermédiaire de canaux (2) d'un bloc de brûleur (3), une réaction à haute température à courte durée de séjour ayant lieu dans la chambre réactionnelle (4) et le mélange réactionnel étant ensuite refroidi rapidement dans une zone de refroidissement rapide (5), **caractérisé en ce que** la transition de la chambre réactionnelle (4) à la zone de refroidissement rapide (5) est réalisée sous la forme d'une fente annulaire qui est de préférence limitée à une largeur de l'ordre de 2 à 200 mm, particulièrement avantageusement de l'ordre de 50 à 150 mm.

4. Réacteur (1) suivant la revendication 3, **caractérisé en ce qu'**on réalise la chambre réactionnelle (4) sous la forme d'une fente annulaire.

5. Réacteur (1) suivant la revendication 3 ou 4, **caractérisé en ce qu'**on oriente les canaux (2) du bloc de brûleur (3) suivant l'axe longitudinal de la chambre réactionnelle (4).

6. Réacteur (1) suivant la revendication 3 ou 4, **caractérisé en ce qu'**on oriente une partie des canaux (2) pour l'amenée du mélange réactionnel et/ou des canaux (6) pour l'amenée d'oxygène additionnel ou d'adjuvants réactionnels sous un angle quelconque par rapport à l'axe longitudinal de la chambre réactionnelle (4) et, pour le reste, on oriente les canaux (2) du bloc de brûleur (3) suivant l'axe longitudinal de la chambre réactionnelle (4).

7. Réacteur (1) suivant l'une des revendications 3 à 6, **caractérisé en ce qu'**on réalise la zone de refroidissement rapide (5) de manière alignée avec la direction de l'axe longitudinal de la chambre réactionnelle (4), avantageusement sous la forme d'une fente, particulièrement avantageusement sous la forme d'une fente annulaire.

8. Réacteur (1) suivant l'une des revendications 3 à 7, **caractérisé en ce qu'**on produit le refroidissement rapide du mélange réactionnel dans la zone de refroidissement rapide (5) par un refroidissement rapide direct ou indirect, le refroidissement rapide direct du mélange réactionnel étant produit dans la zone de refroidissement rapide (5), de préférence par une incorporation en une ou plusieurs étapes d'un milieu réfrigérant dans le mélange réactionnel, particulièrement avantageusement par l'intermédiaire d'un ou de plusieurs distributeurs annulaires.

9. Réacteur (1) suivant la revendication 8, **caractérisé en ce qu'**on produit le refroidissement rapide direct du milieu réactionnel dans la zone de refroidissement rapide réalisée sous la forme d'une fente annulaire (5) par une incorporation directe du milieu réfrigérant dans la zone de refroidissement rapide (5) réalisée sous la forme d'une fente annulaire depuis l'extérieur et/ou depuis l'intérieur.

10. Réacteur (1) suivant l'une des revendications 3 à 9, **caractérisé en ce qu'**on produit dans la zone de refroidissement rapide (5) un refroidissement rapide direct par introduction d'un milieu réfrigérant par des tuyères de refroidissement rapide, qui sont agencées radialement ou tangentiellement par rapport à la direction d'écoulement principal du mélange réactionnel dans le réacteur (1), une mise en place opposée des tuyères de refroidissement rapide étant préférée lors d'une introduction en plusieurs étapes du milieu réfrigérant.

11. Réacteur (1) suivant l'une des revendications 3 à 10, **caractérisé en ce que** toutes les surfaces délimitant la chambre réactionnelle (4) sont réalisées à partir d'une matière céramique réfractaire résistante à une température réactionnelle et comportant une fraction d'oxyde d'aluminium d'au moins 80 % en poids, de préférence d'au moins 95 % en poids, particulièrement avantageusement d'au moins 96 % en poids.

12. Réacteur (1) suivant l'une des revendications 3 à 11, **caractérisé en ce que** la céramique réfractaire est introduite dans la chambre réactionnelle sous la forme de briques ou de blocs ou d'une masse de coulage ou de damage et est ensuite comprimée, séchée et avantageusement cuite par la réaction à haute température.

13. Réacteur (1) suivant l'une des revendications 3 à 12, **caractérisé en ce que** la céramique réfractaire présente une épaisseur de l'ordre de 7 à 30 cm, de préférence une épaisseur de l'ordre de 8 à 10 cm.

14. Utilisation du procédé selon la revendication 1 ou 2 ou du réacteur suivant une des revendications 3 à 13, pour la fabrication d'acétylène par oxydation partielle d'hydrocarbures avec de l'oxygène.
